(19) Europäisches Patentamt
European Patent Office
Office européen des brevets

(11) EP 1 322 664 B1

(12) # EUROPEAN PATENT SPECIFICATION

(45) Date of publication and mention of the grant of the patent:
**12.07.2006 Bulletin 2006/28**

(21) Application number: **01972342.8**

(22) Date of filing: **03.10.2001**

(51) Int Cl.:
***C07K 7/06*** *(2006.01)* ***C07K 7/08*** *(2006.01)*
***G01N 33/542*** *(2006.01)*

(86) International application number:
**PCT/GB2001/004462**

(87) International publication number:
**WO 2002/029407 (11.04.2002 Gazette 2002/15)**

(54) **DYE-LABELLED PEPTIDE AND ITS DIAGNOSTIC USE**

FARBSTOFF-MARKIERTES PEPTID UND DESSEN VERWENDUNG ZUR DIAGNOSTIK

PEPTIDE MARQUE PAR COLORANT ET SON UTILISATION DIAGNOSTIQUE

(84) Designated Contracting States:
**AT BE CH CY DE DK ES FI FR GB GR IE IT LI LU MC NL PT SE TR**

(30) Priority: **04.10.2000 GB 0024351**

(43) Date of publication of application:
**02.07.2003 Bulletin 2003/27**

(73) Proprietor: **GE Healthcare UK Limited**
**Little Chalfont**
**Buckinghamshire HP7 9NA (GB)**

(72) Inventor: **COOK, Neil, D.**
**Princeton, NJ 08540 (US)**

(74) Representative: **Franks, Barry Gerard et al**
**GE Healthcare Limited**
**Amersham Place**
**Little Chalfont,**
**Bucks. HP7 9NA (GB)**

(56) References cited:
**WO-A-00/08054**
**WO-A-00/13026**
**WO-A-97/28261**
**WO-A-99/39203**
**DE-A- 19 812 020**

- **BESSON E.A.: "Synthesis and fluorescent properties of new heterobifunctional fluorescent probes" HETEROCYCLES, vol. 34, no. 2, 1992, pages 273-291, XP008003482 cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 322 664 B1

## Description

The present invention relates to dye-labelled peptides. In particular, the invention relates to new labelling methods whereby single or multiple fluorescent reporter groups may be incorporated into synthetic polypeptide molecules. Processes for the preparation of the fluorescent dye-labelled peptide derivatives are described, together with their applications in assays employing fluorescence detection.

Biochemical assays utilising labelled peptides in which the reporter group may be, for example, a detectable label such as a fluorescent moiety, are well known. Thus, peptides and proteins can be labelled with fluorescent labelling reagents to provide detectable labels for numerous *in vitro* assay procedures. The chemistry of peptide labeling is well documented and a wide range of reagents is available for the chemical modification of peptides. Generally, the choice of labelling reagent and the chemistry of labelling will be determined by the amino acid composition of the molecule to be labelled. Particularly preferred are amine reactive fluorescent labelling reagents and thiol reactive labelling reagents. In the first case, the functional group for labelling is a primary amino group which may be derived from the ε-amino group of lysine, or alternatively the peptide amino-terminus. Particular examples of labelling reagents for ε-amino lysine residues include fluorescein isothiocyanate (FITC), fluorescein N-hydroxysuccinimidyl ester, and the mono- and bis-reactive NHS esters of the cyanine dyes. Although relatively few proteins and peptides have free thiol groups (they generally exist as disulphide groups), thiol labelling procedures have proved very useful for labelling proteins and peptides, using thiol-reactive reagents, for example, iodoacetyl and maleimidyl derivatives of fluorescent molecules. For a review and examples of protein labelling using fluorescent labelling reagents, see "Non-Radioactive Labelling, a Practical Introduction", Garman, A.J. Academic Press,1997; "Handbook of Fluorescent Probes and Research Chemicals", Haugland, R.P., Molecular Probes Inc., 1992).

Two problems may arise in labelling experiments with peptides and in their subsequent use as labelled probes. Firstly, the fluorescent group may be bulky and large in relation to the peptide to be labelled, and may be attached to the peptide via side chain functional groups. As a result, the fluorescent label may often have an adverse effect on the biological activity of the peptide being labelled. This may be a particular problem with the use of long wavelength fluorophores having extended chromophores. Secondly, not all of the amino acids that are generally available in proteins for covalent attachment of a fluorescent group will necessarily be present in peptides.

Besson et al (Heterocycles, 34(2), 273-291 1992) describe heterobifunctional fluorescent compounds bearing an amino group and a carboxylic group. The authors state that in these compounds, the amine function can react with the carboxylic ends of peptides and the carboxylic group can form bridges with side chains of polymers or lateral chains of amino acids in carrier proteins.

There is a continuing need for alternative labelling strategies for biomolecules such as peptides and proteins. Specifically, new methods are required for the introduction of a fluorescent label into a peptide or into a conjugate between a peptide and another biomolecule such as a carbohydrate. The present invention therefore provides new fluorescent reagents and methods which are of use in labelling peptides and proteins as well as other biomolecules possessing suitable functional groups for attachment of the fluorescent label.

Accordingly, the present invention provides a compound comprising a peptide chain containing one or more dye molecules covalently bonded thereto, characterised in that at least one dye molecule is interposed in the amino acid sequence forming the peptide chain such that there is at least one amino acid covalently linked to and on each side of the said at least one dye molecule.

In a first embodiment of the invention, the dye-labelled peptide chain is of the formula (I):

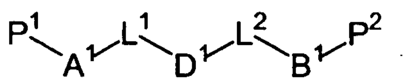

**(I)**

wherein $D^1$ is a dye molecule;

$P^1$ is an amino acid or a sequence comprising at least two amino acids and may include one or more functional groups for reaction with other groups;

$P^2$ is an amino acid or a sequence comprising at least two amino acids and may include one or more functional groups for reaction with other groups;

$A^1$ comprises an atom or a group suitable for attaching $L^1$ to $P^1$ by means of a covalent linkage;

$B^1$ comprises an atom or a group suitable for attaching $L^2$ to $P^2$ by means of a covalent linkage; and

$L^1$ and $L^2$ are each a linker chain and each independently contains from 1-20 linked atoms selected from the group consisting of carbon, nitrogen, oxygen, sulphur and phosphorus and combinations thereof and each $L^1$ and $L^2$ may be independently substituted by one or more groups selected from hydroxyl, halogen, $C_1$-$C_4$ alkyl, $C_1$-$C_4$ alkoxy, aryl, heteroaryl and aralkyl.

In a second embodiment, the dye-labelled peptide chain includes a dye attached to a terminal amino acid of the peptide sequence. In this embodiment, the dye-labelled peptide chain is suitably of the formula (II):

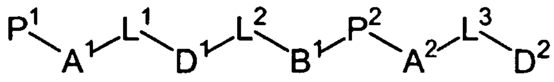

(II)

wherein $D^1$ and $D^2$ may be the same or different and are each a dye molecule;
$P^1$, $P^2$, $A^1$ and $B^1$ are as hereinbefore defined;
$A^2$ comprises an atom or a group suitable for attaching $L^3$ to $P^2$ by means of a covalent linkage; and
$L^1$, $L^2$ and $L^3$ are each as hereinbefore defined for $L^1$ and $L^2$.

In a third embodiment, the dye-labelled peptide chain contains more than one dye interposed in the peptide sequence. In this embodiment, the dye-labelled peptide chain is suitably of the formula (III):

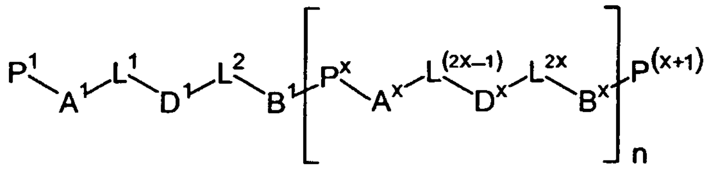

(III)

wherein n is an integer and x is (n + 1); and
$P^1$ to $P^{(x+1)}$, $A^1$ to $A^x$, $B^1$ to $B^x$, $L^1$ to $L^{2x}$ and $D^1$ to $D^x$ are as hereinbefore defined for $P^1$, $P^2$, $A^1$, $B^1$, $L^1$, $L^2$, $L^3$, $D^1$ and $D^2$.

In a further embodiment, the dye-labelled peptide chain containing more than one interposed dye may also include a dye attached to a terminal amino acid.

Suitably, n is an integer from 1 to 5. Preferably, n is 1 or 2.

In a preferred embodiment, the dye-labelled peptide chain contains two dyes interposed in the peptide sequence. In this embodiment, the dye-labelled peptide chain is suitably of the formula (IV):

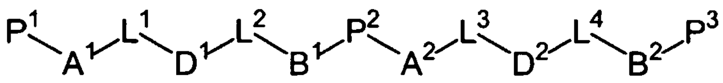

(IV)

wherein $P^1$, $P^2$, $P^3$, $A^1$, $A^2$, $B^1$, $B^2$, $L^1$, $L^2$, $L^3$, $L^4$, $D^1$ and $D^2$ are as hereinbefore defined for $P^1$, $P^2$, $A^1$, $B^1$, $L^1$, $L^2$, $L^3$, $D^1$ and $D^2$.

The term peptide chain as used herein is intended to denote oligopeptides, polypeptides, proteins and fragments thereof.

Suitably, $P^1$ terminates in a functional group for reaction under suitable conditions with $A^1$. Suitably, $P^x$ terminates

in a functional group for reaction under suitable conditions with $B^{(x-1)}$. Alternatively, $P^x$ terminates in a first functional group for reaction under suitable conditions with $B^{(x-1)}$ and in a second functional group for reaction under suitable conditions with $A^x$. Suitably, $P^{(x+1)}$ terminates in a functional group for reaction under suitable conditions with $B^x$.

Suitably, the linker chains $L^1$ to $L^{2x}$ may be selected from linear or branched $C_{1-20}$ alkyl chains, which may optionally contain one or more ether linkages, one or more amide linkages, one or more unsaturated groups, including $-CR^a=CR^a-$, $-C\equiv C-$, and phenylene which may be substituted with 1,2,3 or 4 substituents independently selected from hydroxyl, halogen, $C_1$-$C_4$ alkyl, $C_1$-$C_4$ alkoxy, aryl, heteroaryl and aralkyl and $R^a$ is selected from hydrogen and $C_1$-$C_4$ alkyl. Preferably the linker chains $L^1$ to $L^{2x}$ may be selected from the group consisting of a straight or branched $C_{1-20}$ alkyl chain, a $C_{2-20}$ monoether or polyether and a $C_{2-20}$ atom chain containing up to two secondary amide linkages.

Aryl is an aromatic substituent, for example phenyl or naphthyl, which may be optionally and independently substituted by one or more groups selected from hydroxyl, halogen, $C_1$-$C_4$ alkyl and $C_1$-$C_4$ alkoxy.

Heteroaryl is a mono- or bicyclic 5-10 membered aromatic ring system containing at least one and no more than 3 heteroatoms which may be selected from N, O and S. The heteroaryl may be optionally and independently substituted by one or more groups selected from hydroxyl, halogen, $C_1$-$C_4$ alkyl and $C_1$-$C_4$ alkoxy.

Aralkyl is a $C_1$-$C_4$ alkyl group substituted by an aryl or heteroaryl group.

Halogen groups are those selected from fluorine, chlorine, bromine and iodine.

In one preferred embodiment, $D^1$ (and/or $D^2$ to $D^x$ if present), is a fluorescent dye. In a second preferred embodiment, one or more of $D^1$ to $D^x$ is a fluorescent dye and the remaining $D^1$ to $D^x$ is a non-fluorescent or quenching dye. Preferably, a fluorescent dye and a non-fluorescent dye are at adjacent dye positions, ie. $D^x$ is fluorescent and one or both of $D^{(x+1)}$ and $D^{(x-1)}$ is a non-fluorescent or quenching dye, wherein x is hereinbefore defined.

Suitably, $D^1$ has attached to it linker chains $L^1$ and $L^2$ for linking $D^1$ respectively to one each of a functional group $A^1$ and a reactive group $B^1$. Suitably, each $D^x$ (if present), has attached to it linker chains $L^{(2x-1)}$ and $L^{2x}$ for linking $D^x$ respectively to at least one functional group $A^x$, and/or reactive group $B^x$, wherein x is hereinbefore defined.

Suitably, groups $A^1$ to $A^x$ are capable of forming covalent linkages with the terminal functional groups respectively of $P^1$ to $P^x$, and groups $B^1$ to $B^x$ are capable of forming covalent linkages with the terminal functional groups respectively of $P^2$ to $P^{(x+1)}$, wherein x is hereinbefore defined.

Suitably, groups $A^1$ to $A^x$ may be the same or different and may be selected from amino, hydroxyl and sulphydryl, including their protected derivatives. Suitable protecting groups for amino, hydroxyl and sulphydryl groups, such as those for use in peptide synthetic methods, are well known to the skilled person. Preferably, each of groups $A^1$ to $A^x$ is an amino group or a protected amino group such as the $N^{\alpha}$-t-butyloxycarbonyl (BOC) group or $N^{\alpha}$-9-fluorenylmethyloxycarbonyl (Fmoc) group. Suitably, groups $B^1$ to $B^x$ may be the same or different and may be selected from a carboxyl (including protected or activated carboxyl groups), isothiocyanate, maleimide, haloacetamide, acid halide, hydrazide, vinylsulphone, dichlorotriazine and phosphoramidite. Preferably, each of groups $B^1$ to $B^x$ is a carboxyl or a protected or activated carboxyl group. Suitable activated carboxyl groups $B^1$ to $B^x$ include succinimidyl ester or sulphosuccinimidyl ester. Suitable protected carboxyl groups are those suitable for peptide synthesis, examples of which will be well known to the skilled person and include ester groups such as t-butyl ester, phenacyl ester and 2,2,2-trichloroethyl ester.

Suitably, the amino acids or amino acid sequences $P^1$ to $P^{(x+1)}$ flanking $D^1$ (and $D^2$ to $D^x$ if present) may be selected from naturally occurring L-amino acids, for example: alanine (Ala or A), arginine (Arg or R), asparagine (Asp or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or Y), tryptophan (Trp or W), tyrosine (Tyr or Y) and valine (Val or V). However, it is to be understood that the amino acids comprising $P^1$ to $P^{x+1}$ are not limited to the examples described above and may be represented by analogues of amino acids, including D-amino acids.

Suitable fluorescent dye moieties $D^1$ to $D^x$ may be selected from fluoresceins, rhodamines, coumarins, derivatives of the bis-pyrromethine boron difluoride dyes, such as 3,3',5,5'-tetramethyl-2,2'-pyrromethene-1,1'-boron difluoride, sold under the trademark BODIPY™ by Molecular Probes Inc. and disclosed in US Patent Nos.4774339, 5187223, 5248782 and 5274113 (Haugland and Kang), and cyanine dyes. Particularly preferred fluorescent dyes $D^1$ to $D^x$ for use in the present invention are cyanine dyes having the general formula (V):

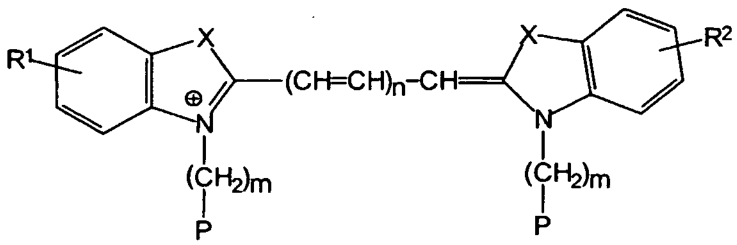

(V)

wherein X is selected from $C(CH_3)_2$, sulphur and oxygen, $R^1$ and $R^2$ are independently selected from the group consisting of $CH_2NH_2$, $SO_3^-$, $CH_2COOH$ and NCS, P is selected from H, $SO_3^-$, $NH_2$ and COOH, n is an integer from 1-3 and m is an integer from 1-5. Cyanine dyes suitable for use in the present invention are disclosed in US Patent No.5268486 (Waggoner et al) and include the CyDyes™: Cy3, Cy3.5, Cy5, Cy5.5 and Cy7. (CyDye and Cy are trademarks of Amersham Pharmacia Biotech UK Limited.)

Additional cyanine dyes are disclosed in PCT Application No.WO 99/31181 (Waggoner et al) and have the general formula (VI):

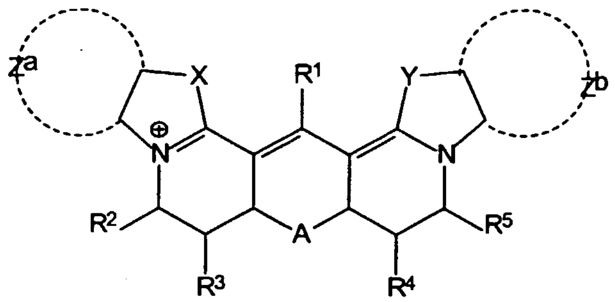

(VI)

optionally substituted by groups $R^2$- $R^9$, wherein groups $R^6$, $R^7$, $R^8$ and $R^9$ are attached to the rings containing X and Y or, optionally are attached to atoms of the $Z^a$ and $Z^b$ ring structures;
$R^2$ to $R^9$ are the same or different and include -$R^{10}$ and -L-$R^{10}$ where $R^{10}$ is selected from neutral groups that reduce water solubility, polar groups that increase water solubility, functional groups that can be used in labelling reactions, reactive groups, electron donating and withdrawing groups that shift the absorption and emission wavelengths of the fluorescent molecule, lipid and hydrocarbon solubilising groups, and L is selected from the group consisting of a straight or branched $C_{1-20}$ alkyl chain, a $C_{2-20}$ monoether or polyether and a $C_{2-20}$ atom chain containing up to four secondary amide linkages;
A is selected from O, S and $NR^{11}$ where $R^{11}$ is the substituted amino radical:

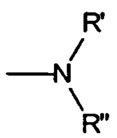

where R' is selected from hydrogen, a $C_{1-4}$ alkyl and aryl and R" is selected from $C_{1-18}$ alkyl, aryl, heteroaryl, an acyl radical having from 2-7 carbon atoms, and a thiocarbamoyl radical;
X and Y may be the same or different and are selected from bis-$C_1$ -$C_4$ alkyl and $C_4$ - $C_5$ spiro alkyl substituted

carbon, oxygen, sulphur, selenium, CH = CH, and N-W wherein N is nitrogen and W is selected from hydrogen, a group $-(CH_2)_nR^{12}$ where n is an integer from 1 to 26 and $R^{12}$ is selected from hydrogen, amino, aldehyde, acetal, ketal, halo, cyano, aryl, heteroaryl, hydroxyl, sulphonate, sulphate, carboxylate, substituted amino, quaternary amino, nitro, primary amide, substituted amide, and groups reactive with amino, hydroxyl, carbonyl, phosphoryl, and sulphydryl groups; and

$Z^a$ and $Z^b$ each represent a bond or the atoms necessary to complete one, two fused or three fused aromatic rings each ring having five or six atoms, selected from carbon atoms and, optionally, no more than two oxygen, nitrogen and sulphur atoms.

In the embodiment of the invention in which the dye-labelled peptide includes at least two different fluorescent dyes, the labelled peptide may exhibit fluorescence resonance energy transfer (FRET) from a fluorescent donor dye to a fluorescent acceptor dye component. Energy transfer occurs between the electronic excited states of two fluorescent dye molecules when they are in sufficient proximity to each other, wherein the excited-state energy of a donor fluorescent dye is transferred to the acceptor dye. For FRET to occur, the wavelength of the emission maximum of the acceptor dye is typically longer than the wavelength of the emission maximum of the donor dye and a portion of the absorption spectrum of the acceptor dye overlaps a portion of the emission spectrum of the donor, for transferring energy absorbed from the donor dye to the acceptor dye. The result is a decrease in the lifetime and a quenching of fluorescence of the donor species and a concomitant increase in the fluorescence intensity of the acceptor species. Energy transfer efficiency depends on several factors such as spectral overlap, spatial separation between donor and acceptor, relative orientation of donor and acceptor molecules, quantum yield of the donor and excited state lifetime of the donor. In a preferred embodiment of the present invention, the fluorescent donor and acceptor dye molecules may be separated in the peptide chain by a distance that provides efficient energy transfer, preferably better than 75%. Closer proximity of the donor and acceptor fluorophores would enhance energy transfer, since efficiency of energy transfer varies as the inverse 6th power of separation of the centres of the chromophores according to Forster's equation:

$$ET \propto K^2 \Phi_D J/R^6 \tau_D$$

where ET is the energy transfer rate constant, $K^2$ is the relative orientation of donor and acceptor transition moments, $\Phi_D$ is the quantum yield of the donor molecule, R is the distance between the centres of the donor and acceptor fluorochromes, J is the overlap between the emission spectrum of the donor and the absorption spectrum of the acceptor fluorochromes, and $\tau_D$ is the excited state lifetime of the donor molecule (Forster, T. "Intermolecular Energy Transfer and Fluorescence", Ann. Physik., Vol.2, p.55, (1948)).

Alternatively, the dye-labelled peptides of the present invention may employ a fluorescent donor dye and a non-fluorescent (or quenching) acceptor dye in an energy transfer relationship. In such a case, the fluorescence emission of the donor is reduced through quenching by the acceptor. When resonance energy transfer is lost through separation of the fluorescent donor dye and the acceptor dye, the fluorescence emission due to the donor dye is restored.

Suitable non-fluorescent (quenching) acceptor species may be selected from 2,4-dinitrophenyl (DNP), 4-(4-dimethylaminophenyl)azobenzoic acid (DABCYL) and non-fluorescent cyanine dyes as described in PCT WO99/64519 (Birch et al). Particularly preferred non-fluorescent acceptor dyes for use in the invention are cyanine dyes having the structure of formula (VII):

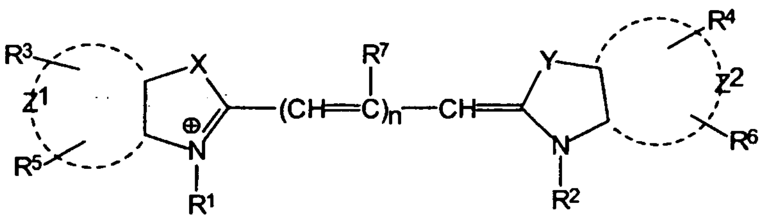

**(VII)**

wherein groups $R^3$, $R^4$, $R^5$ and $R^6$ are attached to the rings containing X and Y or, optionally, are attached to atoms of the $Z^1$ and $Z^2$ ring structures and n is an integer from 1-3;

$Z^1$ and $Z^2$ each represent a bond or the atoms necessary to complete one or two fused aromatic rings each ring having five or six atoms, selected from carbon atoms and, optionally, no more than two oxygen, nitrogen and sulphur

atoms;
X and Y are the same or different and are selected from bis-$C_1$-$C_4$ alkyl- and $C_4$-$C_5$ spiro alkyl-substituted carbon, oxygen, sulphur, selenium, -CH = CH- and N-W wherein N is nitrogen and W is selected from hydrogen, a group -$(CH_2)_mR^8$ where m is an integer from 1 to 26 and $R^8$ is selected from hydrogen, amino, aldehyde, acetal, ketal, halo, cyano, aryl, heteroaryl, hydroxyl, sulphonate, sulphate, carboxylate, substituted amino, quaternary ammonium, nitro, primary amide, substituted amide, and groups reactive with amino, hydroxyl, carbonyl, carboxyl, phosphoryl, and sulphydryl groups;
at least one of groups $R^1$, $R^2$, $R^3$, $R^4$, $R^5$, $R^6$ and $R^7$ is a target bonding group; any remaining groups $R^3$, $R^4$, $R^5$, $R^6$ and $R^7$ groups are independently selected from the group consisting of hydrogen, $C_1$-$C_4$ alkyl, $OR^9$, $COOR^9$, nitro, amino, acylamino, quaternary ammonium, phosphate, sulphonate and sulphate, where $R^9$ is selected from H and $C_1$-$C_4$ alkyl;
any remaining $R^1$ and $R^2$ are selected from $C_1$-$C_{10}$ alkyl which may be unsubstituted or substituted with phenyl the phenyl being optionally substituted by up to two substituents selected from carboxyl, sulphonate and nitro groups;
characterised in that at least one of the groups $R^1$, $R^2$, $R^3$, $R^4$, $R^5$, $R^6$ and $R^7$ comprises a substituent which reduces the fluorescence emission of said dye such that it is essentially non-fluorescent.

Suitably, at least one of the groups $R^3$, $R^4$, $R^5$, $R^6$ and $R^7$ of the non-fluorescent acceptor dyes according to dyes of formula (VII) is a nitro group which may be attached directly to the rings containing X and Y. Alternatively, a mono- or di-nitro-substituted benzyl group may be attached to the rings containing X and Y, which optionally may be further substituted with one or more nitro groups attached directly to the aromatic rings. Preferably, at least one of groups $R^1$, $R^2$, $R^3$, $R^4$, $R^5$, $R^6$ and $R^7$ of the non-fluorescent cyanine dyes of formula (VII) comprises at least one nitro group.

In a preferred embodiment of the invention, the distance R between the centres of $D^1$ and $D^2$ (or $D^x$ and $D^{(x\pm1)}$), where $D^1$ and $D^2$ (or $D^x$ and $D^{(x\pm1)}$) are respectively donor and acceptor dyes in an energy transfer relationship and wherein x is hereinbefore defined, may be from 10 to 80 Angstroms. Thus, in a preferred embodiment, the number of amino acid units in $P^2$ linking $D^1$ and $D^2$ will suitably be between 2 and 20, preferably between 2 and 10. Preferably, the relative orientation of the transition moments of $D^x$ and $D^{(x\pm1)}$, wherein x is hereinbefore defined, during the excited state lifetime of the donor and the proximity of the donor and the acceptor dyes are such that there is sufficient energy transfer.

The fluorescent labelled peptides of the present invention may be prepared by chemical coupling of the fluorescent dye derivatives described above to amino acids or to peptide fragments by techniques well known to the skilled person, for example by means of solid phase peptide synthesis methods as described in "Solid Phase Peptide Synthesis", E. Atherton and R.C.Sheppard, IRL Press 1989. In principle, any amino acid or peptide sequence may be utilised in the formation of fluorescent peptide derivatives of the present invention. The nature and stereochemistry of amino acids utilised in solid phase synthesis techniques is not material to the invention. Rather, the invention discloses the use of fluorescent dye molecules which have been adapted to be suitable for linking to an amino acid or to a peptide chain, and peptides or protein molecules incorporating such dyes. As is known, synthesis of peptides by solid phase techniques is based upon the sequential addition of protected amino acids linked (optionally through a linker group) to a solid phase support. In one commonly employed method, the α-amino group (and side chain amino groups, if any) are suitably protected with acid labile or base labile protecting groups as discussed above. Following addition and coupling of the first amino acid residue, the α-amino protecting group is removed. The chain is extended by the sequential addition of further protected amino acid derivatives or peptide fragments and/or suitably derivatised and protected fluorescent or quencher dye derivatives. In this way, a dye-labelled peptide according to the invention may be constructed by sequential addition of amino acids or a fluorescent dye derivative so as to prepare a peptide containing the desired amino acid sequence, interspersed with one or more fluorescent or quencher dye molecules. Suitably, one (but not both), of the functional group and the reactive group of the dye molecule, as defined hereinbefore, is protected prior to coupling of the dye to the amino-terminus of the preceding amino acid or peptide chain. Once coupled, the protecting group may be removed by methods that are well known to the skilled person. The next protected amino acid or peptide unit is then added using either a coupling reagent or activated amino acid derivative as is known. By this means, a fluorescent dye-labelled peptide derivative of desired amino acid sequence and containing one or more fluorescent dye molecules may be synthesised.

The peptides labelled according to the present invention may be used as fluorescence labels in assays employing fluorescence detection and measurement, for example, by steady state fluorescence intensity, fluorescence lifetime or fluorescence polarisation. Thus, in a further embodiment, there is provided a method for detecting the presence of a biological material which method comprises use of a compound according to formulas (I), (II), (III) or (IV). For example, a fluorescent labelled peptide derivative may be used as probe for a target material, eg. a cell surface receptor, for which the labelled peptide is specific. By this means, the density of cell surface receptors may be determined by the intensity of the fluorescence of the cells when visualised by means of a microscope.

Alternatively, the fluorescence labelled derivative may be used in assay methods involving fluorescence detection. Thus, in a still further embodiment of the present invention, an assay method may comprise separating two components which are in an energy transfer relationship each of said components comprising a peptide or protein or fragment thereof, the first component being labelled with a fluorescent donor dye and the second component being labelled with a non-fluorescent acceptor dye wherein at least one of said dye molecules is interposed in the amino acid chain forming the peptide or protein or fragment such that there is at least one amino acid covalently linked to and on either side of the said at least one dye molecule, and detecting the presence of the first component by measuring emitted fluorescence. Dye-labelled peptides incorporating two such dye moieties may be used in protease assays in which the cleavage of a peptide or protein by a protease is detected by a change in fluorescence intensity. In such assays, the enzyme substrate (peptide, or protein or fragment thereof) may include a sequence whose structure combines the fluorescent donor dye molecule with the non-fluorescent or quenching acceptor dye, covalently bound to the peptide substrate at either side of the substrate bond to be cleaved. The substrate joins the fluorescent donor and the acceptor moieties in close proximity and the intrinsic fluorescence of the donor is reduced through quenching by the acceptor due to resonance energy transfer between the pair of dyes. Resonance energy transfer becomes insignificant when the distance between the donor and acceptor moieties is greater than about 100 Angstroms. Cleavage of the substrate by the protease results in the separation between donor and acceptor dyes and concomitant loss of resonance energy transfer. The fluorescence signal of the donor fluorescent dye increases, thereby enabling accurate measurement of the cleavage reaction. It is to be understood that in the present invention, either $D^1$ or $D^2$ (or $D^x$ or $D^{(x\pm1)}$), may serve as the donor component, the remaining dye $D^1$ or $D^2$ (or $D^x$ or $D^{(x\pm1)}$), being the acceptor. The donor and acceptor species and their position of incorporation into the peptide chain are selected such that proteolytic enzyme cleavage of the substrate is not affected to any significant degree. Such assays may be used in high throughput screening applications, including those in which compounds are to be screened for their inhibitory effects, potentiation effects, agonistic, or antagonistic effects on the reaction under investigation.

Briefly, an assay for the detection of proteolytic enzyme activity may be configured as follows. A reaction mixture is prepared by combining a protease enzyme and a peptide substrate according to the present invention which combines a fluorescent donor dye molecule with a quenching dye attached to the substrate at either side of the substrate bond to be cleaved, as described above. A known or a putative protease inhibitor compound may be optionally included in the reaction mixture. Typically the reaction is performed in buffered solution and the reaction is allowed to proceed to completion. The progress of the reaction may be monitored by observing the steady state fluorescence emission due to the fluorescent donor dye, which is recorded using a spectrofluorimeter.

The invention is further illustrated by reference to the following examples and figures.

Figures

Figure 1 illustrates the course of a trypsin cleavage assay of protease substrate (Compound A), compared with a control dual-labelled fluorescent peptide substrate (Compound B) according to Example 3.1.

Figure 2 shows the results after subtraction of no-enzyme blanks.

Figure 3 illustrates an AspN catalysed cleavage of Compound A according to Example 3.2.

Examples

1. Preparation of Cyanine Dye Labelled Protease Substrate: Cy5Q -Asp-Glu-Val-Asp-Arg-Ser-Gly-Ser-Gly-Ser-Cy3-Ala-Leu-Thr-OH (Compound A)

1.1 Preparation of 2-{(E)-3-[5-(carboxymethyl)-3,3-dimethyl-1-(4-sulfobutyl) 1,3-dihydro-2*H*-indol-2-ylidene]-1-propenyl}-5-({[(9*H*-fluoren-9-ylmethoxy)carbonyl]amino}methyl)-3,3-dimethyl-1-(4-sulphobutyl)-3*H*-indolium (Compound 1)

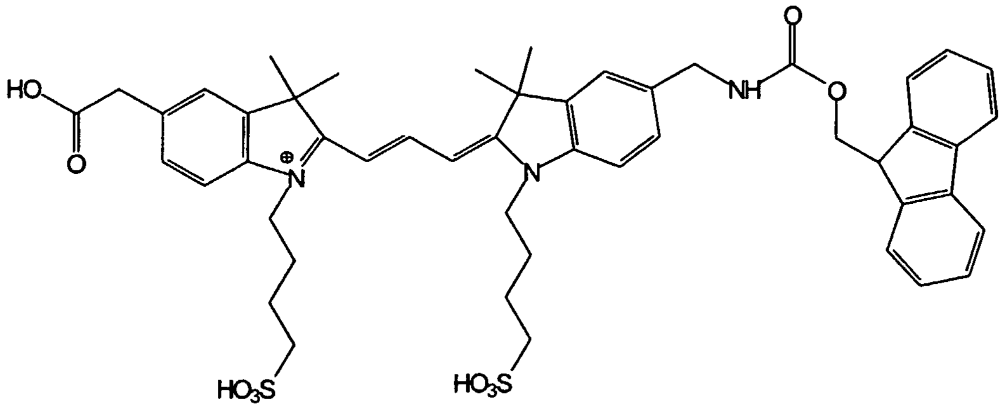

i) 5-[(1,3-Dioxo-1,3-dihydro-2*H*-isoindol-2-yl)methyl]-2,3,3-trimethyl-3*H*-indolenine

To a stirred solution of 2,3,3-trimethylindolenine (20g, 126mmol) in concentrated sulfuric acid (100m1) at RT was added in portions, over forty minutes, N-(hydroxymethyl)phthalimide (20g, 114mmol). The solution was stirred at RT for 70hr. The reaction mixture was poured onto ice (800g) and then made basic by adding concentrated ammonia (~ 300ml) to pH 12. The precipitate which formed was filtered off and washed with water. The product was dried *in vacuo* to give an off white powder (45.05g). MS (MALDI-TOF); found 318($M^+$); [theoretical ($C_{20}H_{18}N_2O_2$) 318].

ii) 5-[(1,3-Dioxo-1,3-dihydro-2*H*-isoindol-2-yl)methyl]-2,3,3-trimethyl-1-(4-sulphobutyl)-3*H*-indolium, inner salt

5-[(1,3-Dioxo-1,3-dihydro-2H-isoindol-2-yl)methyl]-2,3,3-trimethyl-3H-indolenine (5g, 15.7mmol), butyronitrile (80ml) and 1,4-butane sultone (4.3ml, 31.6mmol) were heated together at 120°C for 72hr. The reaction was cooled to room temperature and the product filtered off, washed with butyronitrile (2x50ml) and dried *in vacuo.* The product was obtained as a beige power (4.05g). MS (MALDI-TOF) found 455 ($MH^+$); theoretical ($C_{24}H_{26}N_2O_5S$) 454].

iii) 2,3,3-Trimethyl-3*H*-indol-5-yl-acetic acid

2,3,3-Trimethyl-3H-indol-5-yl-acetic acid was prepared by the method of Southwick *et al,* Org. Prep. Proceed. Int., 20, 274-284 (1989).

iv) 5-(Carboxymethyl)-2,3,3-trimethyl-1-(4-sulphobutyl)-3*H*-indolium, inner salt

2,3,3-Trimethyl-3H-indol-5-yl- acetic acid (5g, 22.1 mmol), butyronitrile (50ml) and 1,4-butane sultone (4.8ml, 35.3mmol) were heated together at 120°C for 72hr. The reaction was cooled to room temperature and the product filtered off, washed with ethyl acetate (2x50ml) and dried *in vacuo.* The product was obtained as a beige powder (4.91 g). MS (MALDI TOF) found 355 ($MH^+$); [theoretical ($C_{17}H_{23}NO_5S$) 354].

v) 2-[(E)-2-Anilinoethenyl]-3,3-dimethyl-5-(1,3-dioxo-1,3-dihydro-2*H*-isoindol-2-yl)-1-(4-sulphobutyl)-3*H*-indolium, salt

5-[(1,3-Dioxo-1,3-dihydro-2*H*-isoindol-2-yl)methyl]-2,3,3-trimethyl-1-(4-sulphobutyl)-3*H*-indolium, inner salt (5g, 9mmol), N,N-diphenylformamidine (2.75g, 14mmol) and acetic acid (50ml) were heated together at 140°C for 18hr. The reaction was allowed to cool to room temperature. The product was purified by HPLC (Dynamax C18 column (50 x 4.14cm); flow rate 25ml/min; gradient of 0 to 100% B over 90 mins (eluent A = 0.1 % TFA in water and eluent B = 0.1 % TFA in acetonitrile); detection at 450nm), the retention time of the product was 65 mins. The fractions containing the desired product were pooled and the solvent removed under reduced pressure. The product was obtained as an orange solid (1.69g). MS (MALDI TOF) found 558 ($M^+$); [theoretical ($C_{31}H_{31}N_3O_5S$) 558].

vi) 2-{(E)-3-[5-(1,3-Dioxo-1,3-dihydro-2*H*-isoindol-2-yl)methyl)-3,3-dimethyl-1-(4-sulphobutyl) 1,3-dihydro-2*H*-indol-2-ylidene]-1-propenyl}-5-(carboxymethyl)-3,3-dimethyl-1-(4-sulphobutyl)-3*H*-indolium, salt

5-(Carboxymethyl)-2, 3, 3-trimethyl-1-(4-sulphobutyl)-3 H-indolium, inner salt (0.5g, 1.41 mmol), 2-[(E)-2-anilinoethenyl]-3,3-dimethyl-5-(1,3-dioxo-1,3-dihydro-2*H*-isoindol-2-yl)-1-(4-sulphobutyl)-3*H*-indolium, salt (0.5g), acetic acid (8ml) pyridine (8ml) and acetic anhydride (2ml) were stirred together at room temperature for 24hr. The reaction

solvents were removed under reduced pressure and the residue purified by HPLC (Dynamax C18 column (50 x 4.14cm); flow rate 25ml/min; gradient of 0 to 100% B over 90 mins (eluent A = 0.1 % TFA in water and eluent B = 0.1 % TFA in acetonitrile); detection at 550nm), the retention time of the product was 70 mins. The fractions containing the desired product were pooled and the solvent removed under reduced pressure. The product was obtained as a magenta solid (505mg). MS (MALDI TOF) found 818 ($M^+$); [theoretical ($C_{42}H_{47}N_3O_{10}S_2$) 818].

vii) 2-{(E)-3-[5-(Aminomethyl)-3,3-dimethyl-1-(4-sulphobutyl)-1,3-dihydro-2*H*-indol-2-ylidene]-1-propenyl}-5-(carboxymethyl)-3,3-dimethyl-1-(4-sulphobutyl)-3*H*-indolium, salt

2-{(E)-3-[5-(1,3-Dioxo-1,3-dihydro-2H-isoindol-2-yl)methyl)-3,3-dimethyl-1-(4-sulphobutyl)-1'2-dihydro-2H-indol-2-ylidene]-1-propenyl}-5-(carboxymethyl)-3-dimethyl-1-(4-sulphobutyl)-3H-indolium, salt (505mg, 0.9mmol) and concentrated hydrochloric acid (25ml) were heated together at 110°C for 20 hrs. The reaction mixture was cooled to room temperature and the solvent removed under reduced pressure. The residue was purified by HPLC (Dynamax C18 column (50 x 4.14cm); flow rate 25ml/min; gradient of 0 to 100% B over 90 mins (eluent A = 0.1 % TFA in water and eluent B = 0.1 % TFA in acetonitrile); detection at 550nm), the retention time of the product was 62 mins. The fractions containing the desired product were pooled and the solvent removed under reduced pressure. The product was obtained as a magenta solid (285mg). MS (MALDI TOF) found 688 ($M^+$); [theoretical ($Ca_4H_4sNaOsS_2$) 689].

viii) 2-{(E)-3-[5-(Carboxymethyl)-3,3-dimethyl-1-(4-sulphobutyl)1,3-dihydro-2*H*-indol-2-ylidenel- 1 -propenyl}-5-({[(9*H*-fluoren-9-ylmethoxy)carbonyl]amino}methyl)-3,3-dimethyl-1-(4-sulphobutyl)-3*H*-indolium, salt (Compound 1)

2-{(E)-3-[5-(Aminomethyl)-3,3-dimethyl-1-(4-sulphobutyl)-1,3-dihydro-2*H*-indol-2-ylidene]-1-propenyl}-5-(carboxymethyl)-3,3-dimethyl-1-(4-sulphobutyl)-3*H*-indolium, salt (200mg, 0.29mmol) was dissolved in dimethylsulphoxide (5ml). N-(9-fluorenylmethoxycarbonyloxy)-succinimide (250mg, 0.74mmol) and diisopropylethylamine (268µl, 1.7mmol) were added and the reaction mixture was stirred at room temperature for 16hrs. Dimethylsulphoxide was removed by washing with diethyl ether to give a magenta residue. The residue was re-dissolved in 10% acetonitrile/water and purified by HPLC (Waters Spherisorb S5ODS2 column (20 x 250mm); flow rate 6ml/min; gradient of 15 to 100% B over 60 mins (eluent A = 0.1 % TFA in water and eluent B = 0.1 % TFA in acetonitrile); detection at 550nm). The fractions containing the desired product were pooled and the solvent removed under reduced pressure. The product was obtained as a magenta solid (136mg). MS (MALDI TOF) found 909 ($M^+$); [theoretical ($C_{49}H_{55}N_3O_8S_2$) 910].

1.2 Synthesis of Cyanine Dye Labelled Protease Substrate: Cy5Q -Asp-Glu-Val-Asp-Arg-Ser-Gly-Ser-Gly-Ser-Cy3-Ala-Leu-Thr-OH

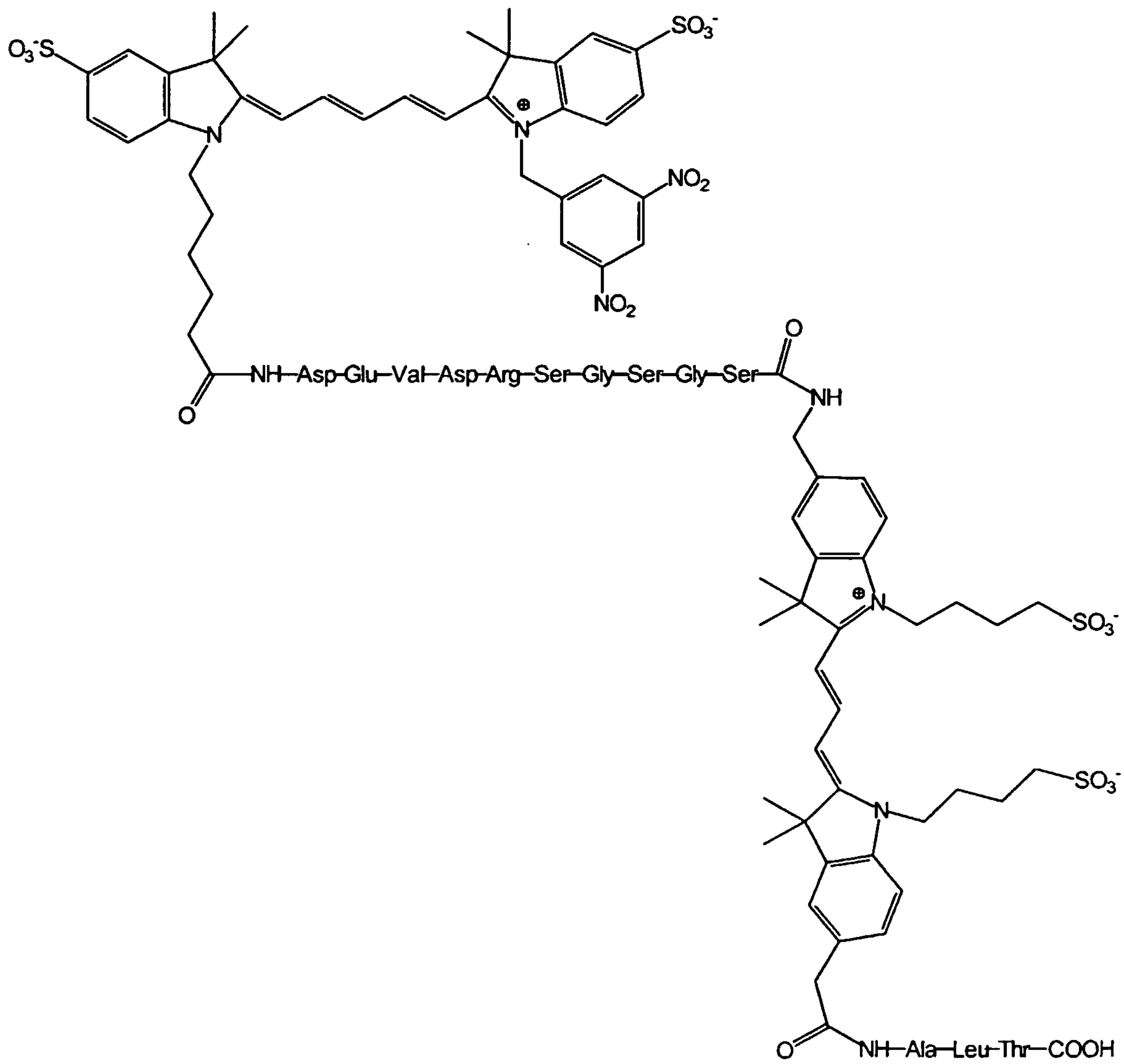

i) Ala-Leu-Thr-Wang Resin

Ala-Leu-Thr-Wang resin was synthesised using a commercially available Perkin-Elmer Model 431 A automated peptide synthesiser and FastMoc™ chemistry, following the instrument manufacturer's recommended procedures throughout. The synthesis was performed on a 0.25 millimolar scale. The resin was removed from the peptide synthesiser and dried *in vacuo.*

ii) Fmoc-Cy3-Ala-Leu-Thr-Wang resin

Fmoc-protected cyanine dye amino acid analogue (Compound 1) (91 mg, 0.1 mmol) was coupled manually to Ala-Leu-Thr-Wang resin (250mg, 0.1 mmol) using-7-azabenzotriazol-1-yloxytris(pyrrolidino)phosphonium-hexafluorophosphate (PyAOP) (62.6mg, 0.12 mmol) and diisopropylethylamine (122µl, 0.7mmol) in N-methyl pyrrolidone (NMP) at room temperature for 16hrs. The reaction solvent was removed and the resin washed with NMP (3 x 10ml). The resin was then capped using a standard capping solution (0.5M acetic anhydride, 0.125M diisopropylethylamine, 0.015M hydroxybenzotriazole), washed with N-methylpyrrolidone, dichloromethane and finally diethyl ether before drying *in vacuo.*

iii) Asp-Glu-Val-Asp-Arg-Ser-Gly-Ser-Gly-Ser-Cy3-Ala-Leu-Thr-Wang resin

Fmoc-Cy3-Ala-Leu-Thr-Wang resin was returned to the Perkin-Elmer Model 431 A automated peptide synthesizer and the final sequence of the peptide built using standard FastMoc™ chemistry, following the instrument manufacturer's recommended procedures throughout. The syntheses were performed on a 0.1 millimolar scale.

iv) Cy5Q-Asp-Glu-Val-Asp-Arg-Ser-Gly-Ser-Gly-Ser-Cy3-Ala-Leu-Thr-OH

2-{5-[1-(5-Carboxypentyl)-3,3-dimethyl-5-sulpho-1,3-dihydro-2*H*-indol-2-ylidene]-1,3-pentadienyl}-1-(3,5-dinitrobenzyl)-3,3-dimethyl-5-sulfo-3*H*-indolium, N-hydroxysuccinimidyl ester (Cy5Q NHS ester) was prepared according to the methods described in PCT Application No. WO 99/64519. Cy5Q NHS ester was coupled to Asp-Glu-Val-Asp-Arg-Ser-Gly-Ser-Gly-Ser-Cy3-Ala-Leu-Thr-Wang resin in DMSO containing diisopropylethylamine at room temperature for 12hrs. The purple resin was filtered off, washed with DMSO, methanol and finally dichloromethane before drying *in vacuo.* The peptide was cleaved from the solid phase using a mixture of 95% trifluoroacetic acid : 2.5% water : 2.5% triisopropylsilane. The crude peptide obtained from the cleavage reaction was purified by conventional C-18 reverse phase HPLC using a linear gradient of water/acetonitrile (both containing 0.1% trifluoroacetic acid). After purification, the peptide was lyophilised to give a purple fluffy solid. The molecular weight of the purified peptide was verified by mass spectrometry analysis. MS (MALDI TOF) found 2753.1 ($M^+$) [theoretical 2752.04].

2. Preparation of Cy5Q-Asp-Glu-Val-Asp-Arg-Ser-Gly-Ser-Gly-Ser-Lys(Cy3)-Ala-Leu-Thr-OH (Compound B)

A dual-labelled peptide was synthesised as a control, using analogous methods.

2.1 Preparation of α-Fmoc-lysine(ε-Cy3)-OH (Compound 2)

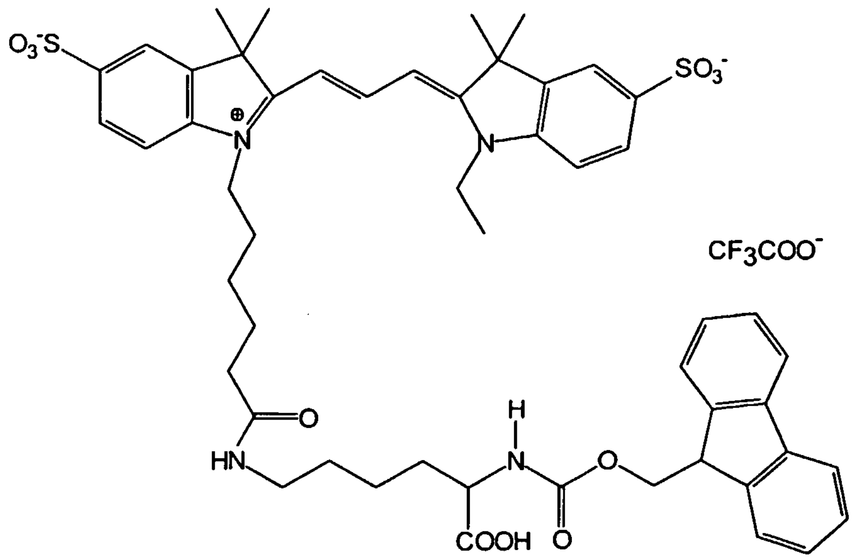

Cy3 mono free acid potassium salt (obtained from Amersham Pharmacia Biotech Ltd) (60mg, 0.095mmol) was dissolved in anhydrous dimethylsulphoxide (2ml). To this was added O-(N-succinimidyl)-N,N,N',N'-bis(tetramethylene)-uronium hexafluorophosphate (100mg, 0.24mmol) and N,N-diisopropylethylamine (80μl). The reaction mixture was stirred at room temperature for 2 hours after which time negligible starting material remained by TLC ($RPC_{18}$, 1:1 methanol:water). The reaction mixture was slowly poured into diethyl ether to precipitate the product, Cy3 NHS ester, which was filtered off and dried *in vacuo.* The product was re-dissolved in anhydrous dimethylsulphoxide (2ml) and N, N-diisopropylethylamine (80μl) added. Fmoc-lysine-OH (50mg, 0.14mmol) was suspended in phosphate buffer (2ml) and the suspension slowly added to the solution of Cy3 NHS ester. The reaction mixture was stirred at room temperature for 2 hours. TLC ($RPC_{18}$, 2:3 methanol:water) showed the disappearance of starting material and the formation of a new product spot. The product was purified by HPLC (Vydac protein peptide $C_{18}$ column (250 x 22.2mm); flow rate 6ml/min; gradient of 15 to 100% B over 60 mins (eluent A = 0.1 % TFA in water and eluent B = 0.1 % TFA in acetonitrile); dual wavelength detection at 254 and 550nm), the retention time of the product was 27 mins. The fractions containing the

desired product were pooled and the solvent removed under reduced pressure. The product was obtained as a magenta solid (70mg). MS (MALDI TOF) found 979 ($M^+$); [theoretical ($C_{52}H_{59}N_4O_{11}S_2$) 980].

2.2 Cy5Q-Asp-Glu-Val-Asp-Arg-Ser-Gly-Ser-Gly-Ser-Lys(Cy3)-Ala-Leu-Thr-OH (Compound B)

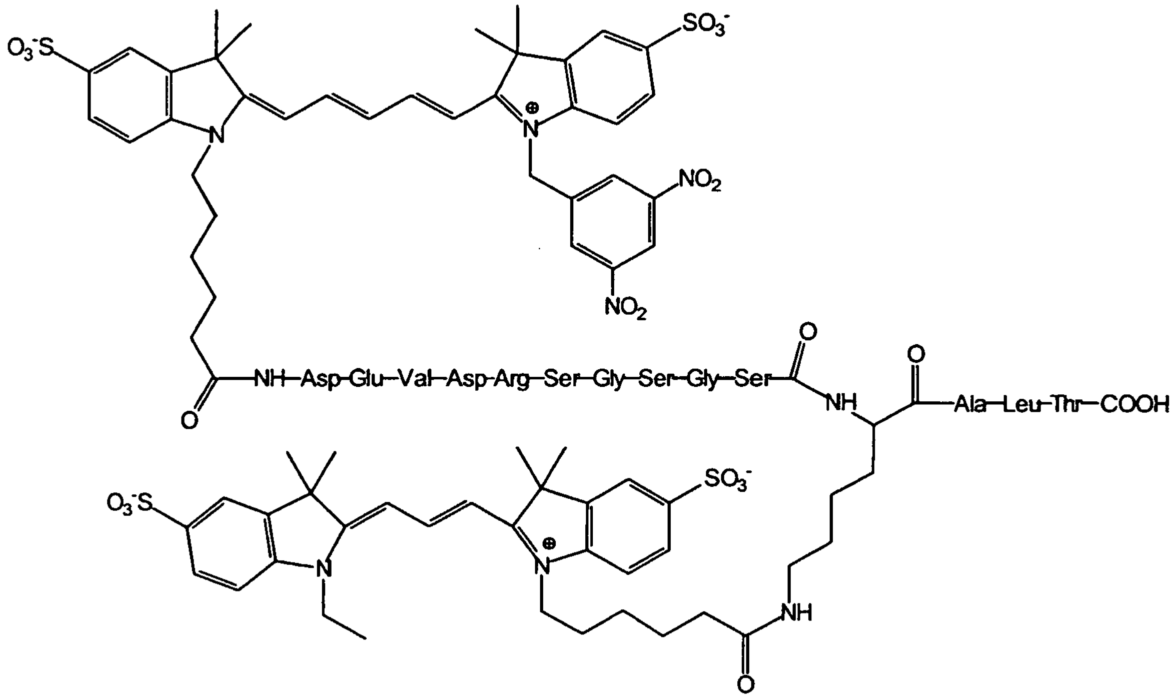

i) Ala-Leu-Thr-Wang Resin

Ala-Leu-Thr-Wang resin was synthesised using a commercially available Perkin-Elmer Model 431 A automated peptide synthesiser and FastMoc™ chemistry, following the instrument manufacturer's recommended procedures throughout. The synthesis was performed on a 0.25 millimolar scale. The resin was removed from the peptide synthesizer and dried *in vacuo.*

ii) Fmoc-Lys(Cy3)-Ala-Leu-Thr-Wang resin

α-Fmoc-lysine(s-Cy3)-OH (Compound 2) (98mg, 0.1 mmol) was coupled manually to Ala-Leu-Thr-Wang resin (250mg, 0.1 mmol) using 7-azabenzotriazol-1-yloxytris(pyrrolidino)phosphonium-hexafluorophosphate (PyAOP) (62.6mg, 0.12 mmol) and diisopropylethylamine (122μl), 0.7mmol) in N-methyl pyrrolidone (NMP) at room temperature for 16hrs. The reaction solvent was filtered off and the resin washed with NMP (3 x 10ml). The resin was then capped using a standard capping solution (0.5M acetic anhydride, 0.125M diisopropylethylamine, 0.015M hydroxybenzotriazole) washed with N-methylpyrrolidone, dichloromethane and finally diethyl ether before drying *in vacuo.*

iii) Asp-Glu-Val-Asp-Arg-Ser-Gly-Ser-Gly-Ser-Lys(Cy3)-Ala-Leu-Thr-Wang resin

Fmoc-Lys(Cy3)-Ala-Leu-Thr-Wang resin was returned to the Perkin-Elmer Model 431 A automated peptide synthesiser and the final sequence of the peptide built using standard FastMoc™ chemistry, following the instrument manufacturer's recommended procedures throughout. The syntheses were performed on a 0.1 millimolar scale.

iv) Cy5Q-Asp-Glu-Val-Asp-Arg-Ser-Gly-Ser-Gly-Ser-Lys(Cy3)-Ala-Leu-Thr-OH (Compound B)

Cy5Q-NHS was coupled to Asp-Glu-Val-Asp-Arg-Ser-Gly-Ser-Gly-Ser-Lys(Cy3)-Ala-Leu-Thr-Wang resin in DMSO containing diisopropylethylamine at room temperature for 12 hrs. The purple resin was then filtered off, washed

with DMSO, methanol and finally dichloromethane before drying *in vacuo.* The peptide was cleaved from the solid phase using a mixture of 95 % trifluoroacetic acid : 2.5 % water : 2.5 % triisopropylsilane. The crude peptide obtained from the cleavage reaction was purified by conventional C-18 reverse phase HPLC using a linear gradient of water/acetonitrile (both containing 0.1 % trifluoroacetic acid). After purification, the peptide was lyophilised to give a purple fluffy solid. The molecular weight of the purified peptide was verified by mass spectrometry analysis.

3. Assay for Protease Enzymes

3.1 Trypsin Cleavage Assay

The protease substrate (Compound A: Cy5Q-Asp-Glu-Val-Asp-Arg-Ser-Gly-Ser-Gly-Ser-Cy3-Ala-Leu-Thr-OH) and a control dual labelled peptide (Compound B: Cy5Q-Asp-Glu-Val-Asp-Arg-Ser-Gly-Ser-Gly-Ser-Lys(Cy3)-Ala-Leu-Thr-OH) were diluted with PBS/0.005% Tween™ 20 to 2μM. To 500μl of each substrate, 1.5 units trypsin (20μl volume) were added. The fluorescence intensity of 100μl volumes (triplicate wells) in a black 96-well microplate (Dynex) was measured at intervals on a fluorescence plate reader using filter sets appropriate for Cy3 (535/10nm for excitation and 569/10nm for emission). Signals from 'No enzyme' blank samples (using 20μl buffer in place of trypsin) were similarly recorded. The results are shown in figure 1.

Figure 2 shows the results after subtraction of the 'no-enzyme' blanks. Protease-catalysed hydrolysis of each substrate results in an increase in Cy3 signal, as the quenching effect of Cy5Q is reduced.

3.2 Endoproteinase AspN Cleavage Assay

The fluorescence intensity of compound A (2μM, 500μl volume) in a quartz cuvette was recorded using a spectrofluorimeter. The sample was excited at 520nm and emission over 540 - 750nm recorded. AspN (100ng, 50μl volume) was added to the sample in the cuvette, which was stoppered, and kept dark at ambient temperature overnight. The fluorescence intensity was again recorded. The result is shown in figure 3. AspN-catalysed hydrolysis of Compound A shows an increase in Cy3 fluorescence, due to cleavage of the substrate, as the quenching effect of Cy5Q is reduced. Tween™ is a trademark of ICI Americas, Inc.

**Claims**

**1.** A compound having the formula:

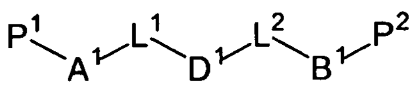

wherein $D^1$ is a fluorescent dye selected from the group consisting of fluoresceins, rhodamines, coumarins, bis-pyrromethine boron difluoride dyes and cyanine dyes;
$P^1$ is an amino acid or a sequence comprising at least two amino acids and may include one or more functional groups for reaction with other groups;
$P^2$ is an amino acid or a sequence comprising at least two amino acids and may include one or more functional groups for reaction with other groups;
$A^1$ comprises an atom or a group suitable for attaching $L^1$ to $P^1$ by means of a covalent linkage;
$B^1$ comprises an atom or a group suitable for attaching $L^2$ to $P^2$ by means of a covalent linkage; and
$L^1$ and $L^2$ are each a linker chain and each independently contains from 1-20 linked atoms selected from linear or branched $C_{1-20}$ alkyl chains, which may optionally contain one or more ether linkages, one or more amide linkages, one
or more unsaturated groups, including $-CR^a=CR^a-$, $-C\equiv C-$, and phenylene which may be substituted with 1,2,3 or 4 substituents independently selected from hydroxyl, halogen, $C_1$-$C_4$ alkyl, $C_1$-$C_4$ alkoxy, aryl, heteroaryl and aralkyl and $R^a$
is selected from hydrogen and $C_1$-$C_4$ alkyl.

**2.** A compound having the formula:

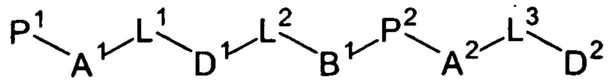

wherein $D^1$ and $D^2$ may be the same or different and are each a dye molecule selected from:

i) a fluorescent dye selected from the group consisting of fluoresceins, rhodamines, coumarins, bis-pyrromethine boron difluoride dyes and cyanine dyes, or
ii) a non-fluorescent (quenching) acceptor dye selected from 2,4-dinitrophenyl (DNP), 4-(4-dimethylaminophenyl)azobenzoic acid (DABCYL) and non-fluorescent cyanine dyes;

$P^1$, $P^2$, $A^1$ and $B^1$ are as hereinbefore defined in claim 1;
$A^2$ comprises an atom or a group suitable for attaching $L^3$ to $P^2$ by means of a covalent linkage; and
$L^1$, $L^2$ and $L^3$ are each as hereinbefore defined in claim 1 for $L^1$ and $L^2$.

**3.** A compound having the formula:

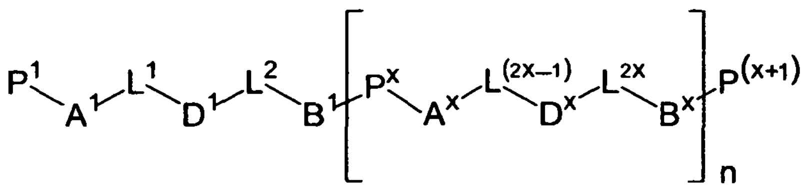

wherein:

n is an integer and x is (n+1); and either,
$D^1$ and/or $D^2$ to $D^x$ is a fluorescent dye
or,
one or more of $D^1$ to $D^x$ is a fluorescent dye and the remaining $D^1$ to $D^x$ is a non-fluorescent or quenching dye; wherein said fluorescent dye is selected from the group consisting of fluoresceins, rhodamines, coumarins, bis-pyrromethine boron difluoride dyes and cyanine dyes; and
said non-fluorescent (quenching) acceptor dye is selected from 2,4-dinitrophenyl (DNP), 4-(4-dimethylaminophenyl)azobenzoic acid (DABCYL) and non-fluorescent cyanine dyes; and
$P^1$ to $P^{(x+1)}$, $A^1$ to $A^x$, $B^1$ to $B^x$, $L^1$ to $L^{2x}$ and $D^1$ to $D^x$ are as hereinbefore defined in claim 2 for $P^1$, $P^2$, $A^1$, $B^1$, $L^1$, $L^2$, $L^3$, $D^1$ and $D^2$.

**4.** A compound according to claim 3 further including a dye attached to a terminal amino acid and wherein said dye is selected from:

i) a fluorescent dye selected from the group consisting of fluoresceins, rhodamines, coumarins, bis-pyrromethine boron difluoride dyes and cyanine dyes, or
ii) a non-fluorescent (quenching) acceptor dye selected from 2,4-dinitrophenyl (DNP), 4-(4-dimethylaminophenyl)azobenzoic acid (DABCYL) and non-fluorescent cyanine dyes.

**5.** A compound according to claim 3 having the formula:

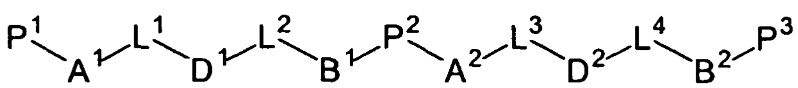

wherein $P^1$, $P^2$, $P^3$, $A^1$, $A^2$, $B^1$, $B^2$, $L^1$, $L^2$, $L^3$, $L^4$, $D^1$ and $D^2$ are as hereinbefore defined in claim 2 for $P^1$, $P^2$, $A^1$, $B^1$, $L^1$, $L^2$, $L^3$, $D^1$ and $D^2$.

**6.** A compound according to claims 2 to 5 wherein the distance R between the centres of $D^1$ and $D^2$ (or $D^x$ and $D^{(x\pm1)}$), where $D^1$ and $D^2$ (or $D^x$ and $D^{(x\pm1)}$) are respectively donor and acceptor dyes in an energy transfer relationship and wherein x is hereinbefore defined in claim 3, may be from 10 to 80 Angstroms.

**7.** A compound according to claim 6 wherein the relative orientation of the transition moments of $D^x$ and $D^{(x\pm1)}$ during the excited state lifetime of the donor and the proximity of the donor and the acceptor dyes are such that there is sufficient energy transfer.

**8.** A compound according to claims 2 to 7 wherein $D^1$ and/or $D^2$ to $D^x$ is a fluorescent dye, wherein x is hereinbefore defined in claim 3.

**9.** A compound according to claims 2 to 7 wherein one or more of $D^1$ to $D^x$ is a fluorescent dye and the remaining $D^1$ to $D^x$ is a non-fluorescent (quenching) dye, wherein x is hereinbefore defined in claim 3.

**10.** A compound according to claim 9 wherein said fluorescent dye and said non-fluorescent dye are at adjacent positions.

**11.** An in vitro method for detecting the presence of a biological material which method comprises use of a compound according to any one of claims 1 to 10.

**12.** An assay method which comprises:

a) separating two components which are in an energy transfer relationship each of said components comprising a peptide chain, the first component being labelled with a fluorescent donor dye and the second component being labelled with a non-fluorescent acceptor dye wherein at least one of said dye molecules is interposed in the amino acid sequence forming the peptide chain such that there is at least one amino acid covalently linked to and on each side of the said at least one dye molecule; and
b) detecting the presence of the first component by measuring emitted fluorescence;

wherein said fluorescent donor dye is selected from the group consisting of fluoresceins, rhodamines, coumarins, bis-pyrromethine boron difluoride dyes and cyanine dyes, and
said non-fluorescent (quenching) acceptor dye is selected from 2,4-dinitrophenyl (DNP), 4-(4-dimethylaminophenyl) azobenzoic acid (DABCYL) and non-fluorescent cyanine dyes.

**13.** A method according to claim 12 wherein said assay method is a proteolytic enzyme cleavage assay.

**14.** Use of a compound according to any one of claims 1-10 for analysis or detection.

## Patentansprüche

**1.** Verbindung mit der Formel:

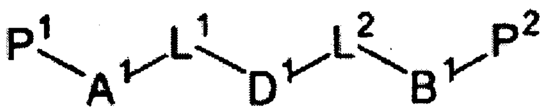

wobei $D^1$ ein Fluoreszenzfarbstoff ist, ausgewählt aus der Gruppe, die besteht aus Fluoresceinen, Rhodaminen, Cumarinen, Bis-pyrromethin-bordifluorid-Farbstoffen und Cyaninfarbstoffen;
$P^1$ für eine Aminosäure oder eine Sequenz steht, die mindestens 2 Aminosäuren umfasst und eine oder mehrere funktionelle Gruppen für eine Reaktion mit anderen Gruppen enthalten kann;
$P^2$ für eine Aminosäure oder eine Sequenz steht, die mindestens 2 Aminosäuren umfasst und eine oder mehrere

funktionelle Gruppen für eine Reaktion mit anderen Gruppen enthalten kann;
$A^1$ ein Atom oder eine Gruppe umfasst, die zum Binden von $L^1$ bis $P^1$ mittels einer kovalenten Bindung geeignet ist;
$B^1$ ein Atom oder eine Gruppe umfasst, die zum Binden von $L^2$ bis $P^2$ mittels einer kovalenten Bindung geeignet ist;
$L^1$ und $L^2$ jeweils für eine Linkerkette stehen und jeweils unabhängig 1-20 gebundene Atome enthalten, die ausgewählt sind aus linearen oder verzweigten $C_{1-20}$-Alkylketten, die gegebenenfalls eine oder mehrere Etherbindungen, eine oder mehrere Amidbindungen, eine oder mehrere ungesättigte Gruppen, einschließlich $-CR^a=CR^a-$, $-C\equiv C-$ und Phenylen enthalten können, ggf. mit 1, 2, 3 oder 4 Substituenten substituiert, unabhängig ausgewählt aus Hydroxyl, Halogen, $C_1$-$C_4$-Alkyl, $C_1$-$C_4$-Alkoxy, Aryl, Heteroaryl und Aralkyl, und $R^a$ ausgewählt ist aus Wasserstoff und $C_1$-$C_4$-Alkyl.

**2.** Verbindung mit der Formel:

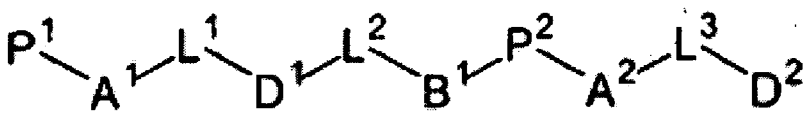

wobei $D^1$ und $D^2$ gleich oder verschieden sein können und jeweils ein Farbstoffmolekül sind, das ausgewählt ist aus:

i) einem Fluoreszenzfarbstoff, ausgewählt aus der Gruppe, die besteht aus Fluoresceinen, Rhodaminen, Cumarinen, Bis-pyrromethinbordifluorid-Farbstoffen und Cyaninfarbstoffen oder
ii) einem nicht fluoreszierenden (löschenden) Akzeptor-Farbstoff, ausgewählt aus 2,4-Dinitrophenyl (DNP), 4-(4-Dimethylaminophenyl)azobenzoesäure (DABCYL) und nicht fluoreszierenden Cyaninfarbstoffen;

$P^1$, $P^2$, $A^1$ und $B^1$ wie hier zuvor in Anspruch 1 definiert sind;
$A^2$ ein Atom oder eine Gruppe umfasst, die zum Binden von $L^3$ bis $P^2$ mittels einer kovalenten Bindung geeignet sind; und
$L^1$, $L^2$ und $L^3$ wie hier zuvor in Anspruch 1 für $L^1$ und $L^2$ definiert sind.

**3.** Verbindung mit der Formel:

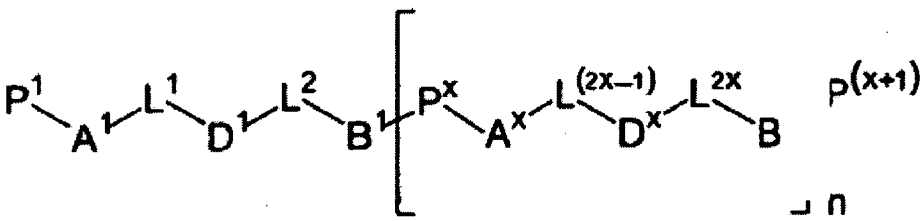

wobei:

n für eine ganze Zahl und x für (n+1) steht; und entweder
$D^1$ und/oder $D^2$ bis $D^x$ ein Fluoreszenzfarbstoff ist oder
einer oder mehrere der Gruppen $D^1$ bis $D^x$ ein Fluoreszenzfarbstoff ist und die verbleibenden $D^1$ bis $D^x$ ein nicht fluoreszierender oder löschender Farbstoff ist;

wobei der Fluoreszenzfarbstoff ausgewählt ist aus der Gruppe, die besteht aus Fluoresceinen, Rhodaminen, Cumarinen, Bis-pyrromethin-bordifluorid-Farbstoffen und Cyaninfarbstoffen;
und der nicht fluoreszierende (löschende) Akzeptorfarbstoff ausgewählt ist aus 2,4-Dinitrophenyl (DNP), 4-(4-Dimethylaminophenyl)azobenzoesäure (DABCYL) und nicht fluoreszierenden Cyaninfarbstoffen;
und $P^1$ bis $P^{(x+1)}$, $A^1$ bis $A^x$, $B^1$ bis $B^x$, $L^1$ bis $L^{2x}$ und $D^1$ bis $D^x$ wie zuvor in Anspruch 2 für $P^1$, $P^2$, $A^1$, $B^1$, $L^1$, $L^2$,

$L^3$, $D^1$ und $D^2$ definiert sind.

**4.** Verbindung nach Anspruch 3, weiter umfassend einen Farbstoff, der an eine terminale Aminosäure gebunden ist, und wobei der Farbstoff ausgewählt ist aus:

i) einem Fluoreszenzfarbstoff ausgewählt aus der Gruppe, die besteht aus Fluoresceinen, Rhodaminen, Cumarinen, Bis-pyrromethinbordifluorid-Farbstoffen und Cyaninfarbstoffen oder
ii) einem nicht fluoreszierenden (löschenden) Akzeptor-Farbstoff, ausgewählt aus 2,4-Dinitrophenyl (DNP), 4-(4-Dimethylaminophenyl)azobenzoesäure (DABCYL) und nicht fluoreszierenden Cyaninfarbstoffen.

**5.** Verbindung nach Anspruch 3 mit der Formel:

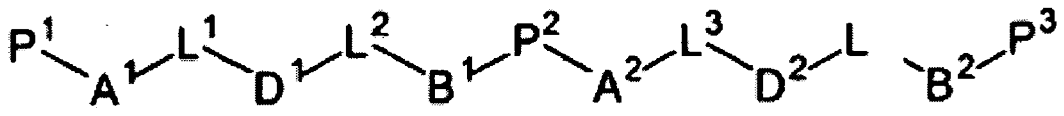

wobei $P^1$, $P^2$, $P^3$, $A^1$, $A^2$, $B^1$, $B^2$, $L^1$, $L^2$, $L^3$, $L^4$, $D^1$ und $D^2$ wie zuvor in Anspruch 2 für $P^1$, $P^2$, $A^1$, $B^1$, $L^1$, $L^2$, $L^3$, $D^1$ und $D^2$ definiert sind.

**6.** Verbindung nach den Ansprüchen 2 bis 5, wobei der Abstand R zwischen den Zentren von $D^1$ und $D^2$ (oder $D^x$ und $D^{(x\pm 1)}$), wobei $D^1$ und $D^2$ (oder $D^x$ und $D^{(x\pm 1)}$) entsprechende Donor- und Akzeptorfarbstoffe in einer Energietransfer-Beziehung sind und wobei x wie zuvor in Anspruch 3 definiert ist, 10 bis 80 Angström betragen kann.

**7.** Verbindung nach Anspruch 6, wobei die relative Orientierung der Übergangsmomente von $D^x$ und $D^{(x\pm 1)}$ während der Lebensdauer des angeregten Zustandes des Donors und die Nähe der Donor- und der Akzeptorfarbstoffe derart sind, dass es einen ausreichenden Energietransfer gibt.

**8.** Verbindung nach den Ansprüchen 2 bis 7, wobei $D^1$ und/oder $D^2$ bis $D^x$ ein Fluoreszenzfarbstoff ist, wobei x wie zuvor in Anspruch 3 definiert ist.

**9.** Verbindung nach den Ansprüchen 2 bis 7, wobei einer oder mehrere der Gruppen $D^1$ bis $D^x$ ein Fluoreszenzfarbstoff ist und das verbleibende $D^1$ bis $D^x$ ein nicht fluoreszierender (löschender) Farbstoff ist, wobei x wie zuvor in Anspruch 3 definiert ist.

**10.** Verbindung nach Anspruch 9, wobei der Fluoreszenzfarbstoff und der nicht fluoreszierende Farbstoff sich an benachbarten Positionen befinden.

**11.** In-vitro-Verfahren zum Detektieren des Vorhandenseins eines biologischen Materials, wobei das Verfahren die Verwendung einer Verbindung nach einem der Ansprüche 1 bis 10 umfasst.

**12.** Assay-Verfahren, umfassend:

a) Trennen zweier Komponenten, die sich in einer Energietransfer-Beziehung befinden, wobei jede der Komponenten eine Peptidkette umfasst, die erste Komponente mit einem Fluoreszenz-Donorfarbstoff markiert ist und die zweite Komponente mit einem nicht-fluoreszierenden Akzeptorfarbstoff markiert ist, wobei mindestens eines der Farbstoffmoleküle in die Aminosäuresequenz, die die Peptidkette bildet, derart eingefügt ist, dass es mindestens eine Aminosäure kovalent gebunden an und auf jeder Seite des mindestens einen Farbstoffmoleküls gibt; und
b) Detektieren des Vorhandenseins der ersten Komponente durch Messen der emittierten Fluoreszenz;

wobei der Fluoreszenz-Donorfarbstoff ausgewählt ist aus der Gruppe, die besteht aus Fluoresceinen, Rhodaminen, Cumarinen, Bis-pyrromethinbordifluorid-Farbstoffen und Cyaninfarbstoffen, und
der nicht fluoreszierende (löschende) Akzeptorfarbstoff ausgewählt ist aus 2,4-Dinitrophenyl (DNP), 4-(4-Dimethylaminophenyl)azobenzoesäure (DABCYL) und nicht fluoreszierenden Cyaninfarbstoffen.

**13.** Verfahren nach Anspruch 12, wobei das Assayverfahren ein proteolytischer Enzymspaltungsassay ist.

**14.** Verwendung einer Verbindung nach einem der Ansprüche 1 bis 10 für eine Analyse oder eine Detektion.

## Revendications

**1.** Composé présentant la formule :

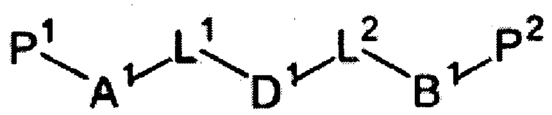

dans lequel $D^1$ est un colorant fluorescent sélectionné à partir du groupe constitué par les fluorescéines, les rhodamines, les coumarines, les colorants à base de bifluorure bis-pyrrométhine de bore et les colorants cyanines ;
$P^1$ est un acide aminé ou une séquence comprenant au moins deux acides aminés et peut comporter un ou plusieurs groupes fonctionnels pouvant réagir avec d'autres groupes ;
$P^2$ est un acide aminé ou une séquence comprenant au moins, deux acides aminés et peut comporter un ou plusieurs groupes fonctionnels pouvant réagir avec d'autres groupes ;
$A^1$ comprend un atome ou un groupe approprié pour lier $L^1$ à $P^1$ au moyen d'une liaison covalente ;
$B^1$ comprend un atome ou un groupe approprié pour lier $L^2$ à $P^2$ au moyen d'une liaison covalente; et
$L^1$ et $L^2$ sont chacun une chaîne d'agent liant et chacun contient, de manière indépendante, de 1 à 20 atomes liés sélectionnés à partir de chaînes alkyle en $C_{1-20}$ linéaires ou ramifiées, qui peuvent, en variante, contenir une ou plusieurs liaisons éther, ou une ou plusieurs liaisons amides, un ou plusieurs groupes insaturés, comprenant $-CR^a=CR^a-$, $-C\equiv C-$, et du phénylène qui peut être substitué avec 1, 2, 3 ou 4 substituants sélectionnés de manière indépendante à partir d'un hydroxyle, un halogène, un alkyle en $C_1$-$C_4$, un alkoxy en $C_1$-$C_4$, un aryle, un hétéroaryle et un aralkyle et $R^a$ est sélectionné à partir de l'hydrogène et d'un alkyle en $C_{1-}C_4$.

**2.** Composé selon la formule :

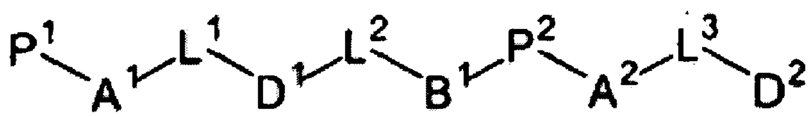

dans lequel $D^1$ et $D^2$ peuvent être identiques ou différents et sont chacun une molécule de colorant sélectionnée à partir de :

i) un colorant fluorescent sélectionné à partir du groupe constitué par les fluorescéines, les rhodamines, les coumarines, les colorants à base de bifluorure bis-pyrrométhine de bore et les colorants cyanines, ou
ii) un colorant receveur non fluorescent (d'étouffement) sélectionné à partir du 2,4-dinitrophényle (DNP), de l'acide 4-(4-diméthylaminophényle)azobenzoïque (DABCYL) et de colorants cyanines non fluorescents ;

$P^1$, $P^2$, $A^1$ et $B^1$ sont tels que définis précédemment dans la revendication 1 ;
$A^2$ comprend un atome ou un groupe approprié afin de lier $L^3$ à $P^2$ au moyen d'une liaison covalente ; et
$L^1$, $L^2$ et $L^3$ sont chacun tels que définis précédemment dans la revendication 1 pour $L^1$ et $L^2$.

**3.** Composé présentant la formule :

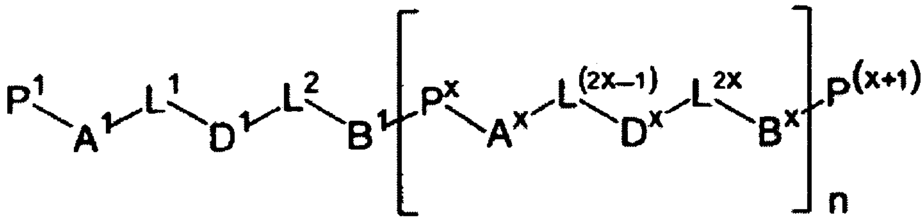

dans lequel :

n est un entier et x est (n+1) ; et soit,
$D^1$ et/ou $D^2$ à $D^x$ représentent un colorant fluorescent
soit,
un ou plusieurs de $D^1$ à $D^x$ représentent un colorant fluorescent et le reste de $D^1$ à $D^x$ représentent un colorant non fluorescent ou modérateur ;
dans lequel ledit colorant fluorescent est sélectionné à partir du groupe constitué par les fluorescéines, les rhodamines, les coumarines, les colorants à base de bifluorure bis-pyrrométhine de bore et les colorants cyanines, et
ledit colorant receveur non fluorescent (modérateur) est sélectionné à partir du 2,4 dinitrophényle (DNP), de l'acide 4-(4-diméthylaminophényle) azobenzoïque (DABCYL) et des colorants cyanines non fluorescents ; et $P^1$ à $P^{(x+1)}$, $A^1$ à $A^x$, $B^1$ à $B^x$, $L^1$ à $L^{2x}$ et $D^1$ à $D^x$ sont tels que définis précédemment dans la revendication 2 pour $P^1$, $P^2$, $A^1$, $B^1$, $L^1$, $L^2$, $L^3$, $D^1$ et $D^2$.

**4.** Composé selon la revendication 3, comprenant, en outre, un colorant lié à une terminaison d'acide aminé et dans lequel ledit colorant est sélectionné à partir de :

i) un colorant fluorescent sélectionné à partir du groupe constitué par les fluorescéines, les rhodamines, les coumarines, les colorants à base de bifluorure bis-pyrrométhine de bore et les colorants cyanines, ou
ii) un colorant receveur non fluorescent (modérateur) sélectionné à partir du 2,4-dinitrophényle (DNP), de l'acide 4-(4-diméthylaminophényle) azobenzoïque (DABCYL) et des colorants cyanines non fluorescents.

**5.** Composé selon la revendication 3, présentant la formule :

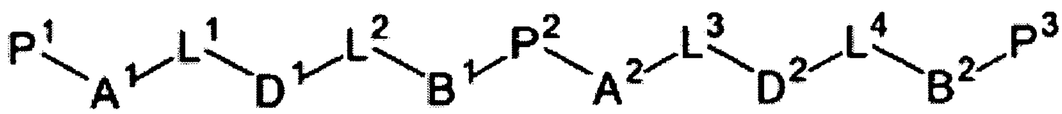

dans lequel $P^1$, $P^2$, $P^3$, $A^1$, $A^2$, $B^1$, $B^2$, $L^1$, $L^2$, $L^3$, $L^4$, $D^1$ et $D^2$ sont tels que définis précédemment dans la revendication 2 pour $P^1$, $P^2$, $A^1$, $B^1$, $L^1$, $L^2$, $L^3$, $D^1$ et $D^2$.

**6.** Composé selon les revendications 2 à 5, dans lequel la distance R entre les centres de $D^1$ et $D^2$ (ou $D^x$ et $D^{(x\pm1)}$), où $D^1$ et $D^2$ (ou $D^x$ et $D^{(x\pm1)}$) sont respectivement des colorants donneur et receveur dans une relation de transfert d'énergie et dans lequel x qui est défini précédemment dans la revendication 3, peut être compris entre 10 et 80 angströms.

**7.** Composé selon la revendication 6, dans lequel l'orientation relative des moments de transition de $D^x$ et $D^{(x\pm1)}$ au cours de la durée de vie à l'état excité du donneur et la proximité des colorants donneurs et receveurs sont tels qu'il se produit un transfert d'énergie suffisant.

**8.** Composé selon les revendications 2 à 7, dans lequel $D^1$ et/ou $D^2$ à $D^x$ est un colorant fluorescent, dans lequel x est tel que défini précédemment dans la revendication 3.

**9.** Composé selon les revendications 2 à 7, dans lequel un ou plusieurs de $D^1$ à $D^x$ représentent un colorant fluorescent et le reste de $D^1$ à $D^x$ représentent un colorant non fluorescent (modérateur), dans lequel x est tel que défini

précédemment dans la revendication 3.

**10.** Composé selon la revendication 9, dans lequel ledit colorant fluorescent et ledit colorant non fluorescent sont à des positions adjacentes.

**11.** Procédé de détection *in vitro* de la présence d'un matériau biologique, lequel procédé comprend l'utilisation d'un composé selon l'une quelconque des revendications 1 à 10.

**12.** Procédé de dosage qui comprend :

a) la séparation de deux composants qui sont en relation de transfert d'énergie, chacun desdits composants comprenant une chaîne peptidique, le premier composant étant marqué avec un colorant donneur fluorescent et le second composant étant marqué avec un colorant receveur non fluorescent dans lequel au moins l'une desdites molécules de colorant est interposée dans la séquence d'acides aminés formant la chaîne peptidique de telle sorte qu'il existe au moins un acide aminé lié de manière covalente à ladite au moins une molécule de colorant et de chaque côté de celle-ci ; et
b) la détection de la présence du premier composant en mesurant la fluorescence émise ;

dans lequel ledit colorant donneur fluorescent est sélectionné à partir du groupe constitué par les fluorescéines, les rhodamines, les coumarines, les colorants au bifluorure bis-pyrrométhine de bore et les colorants cyanines, et ledit colorant receveur non fluorescent (modérateur) est sélectionné à partir du 2,4 dinitrophényle (DNP), de l'acide 4-(4-diméthylaminophényle) azobenzoïque (DABCYL) et des colorants cyanines non fluorescents.

**13.** Procédé selon la revendication 12, dans lequel ledit procédé de dosage est un dosage de clivage d'enzyme protéolytique.

**14.** Utilisation d'un composé selon l'une quelconque des revendications 1 à 10 pour l'analyse ou la détection.

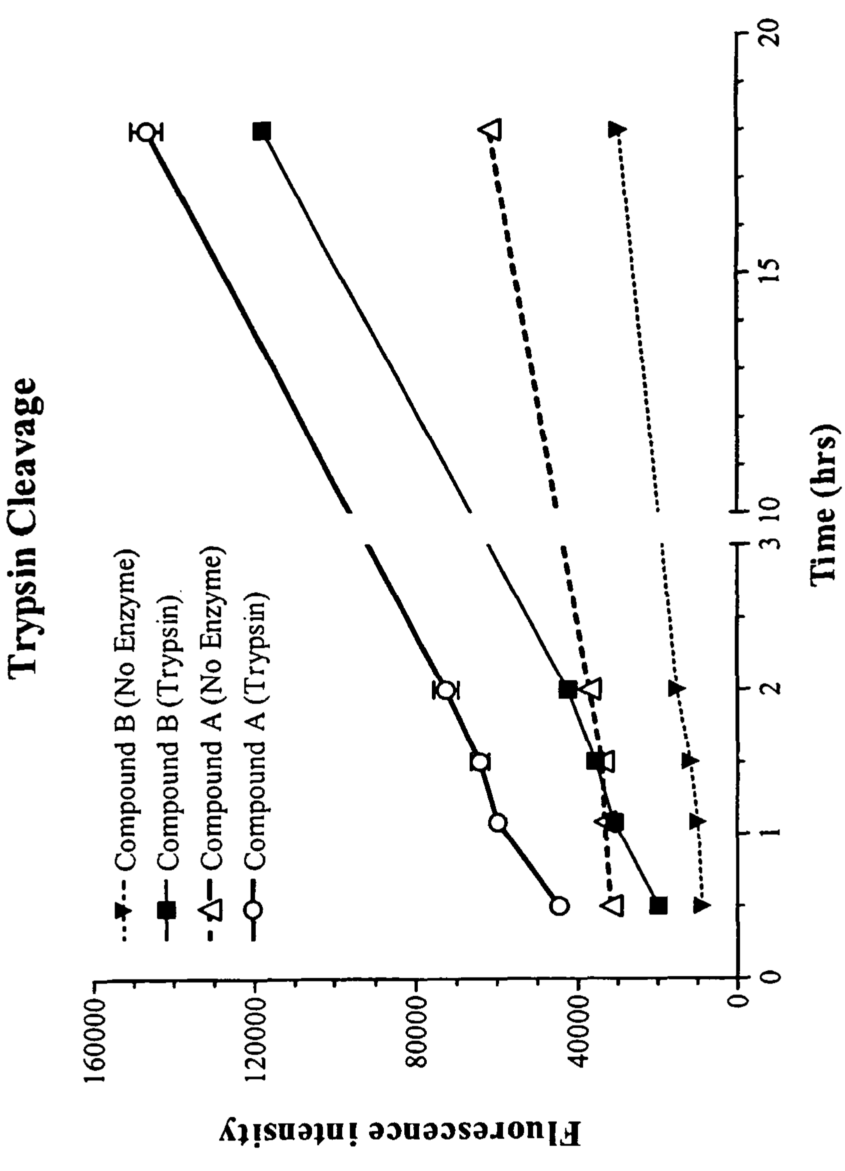
Trypsin Cleavage
Compound B (No Enzyme)
Compound B (Trypsin)
Compound A (No Enzyme)
Compound A (Trypsin)
Fluorescence intensity
160000
120000
80000
40000
0
0
1
2
3 10
15
20
Time (hrs)

Figure 1

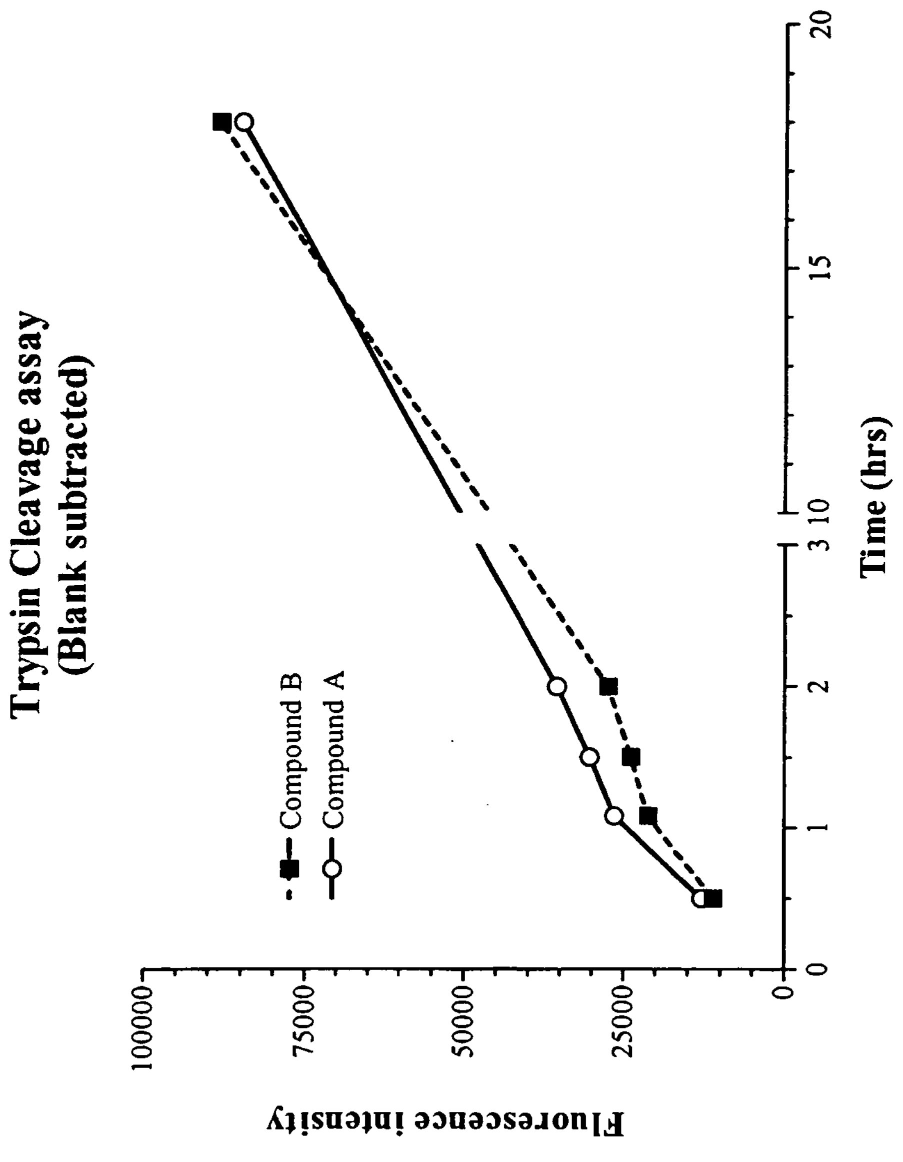
Trypsin Cleavage assay
(Blank subtracted)
Compound B
Compound A
Fluorescence intensity
100000
75000
50000
25000
0
0
1
2
3 10
15
20
Time (hrs)

Figure 2

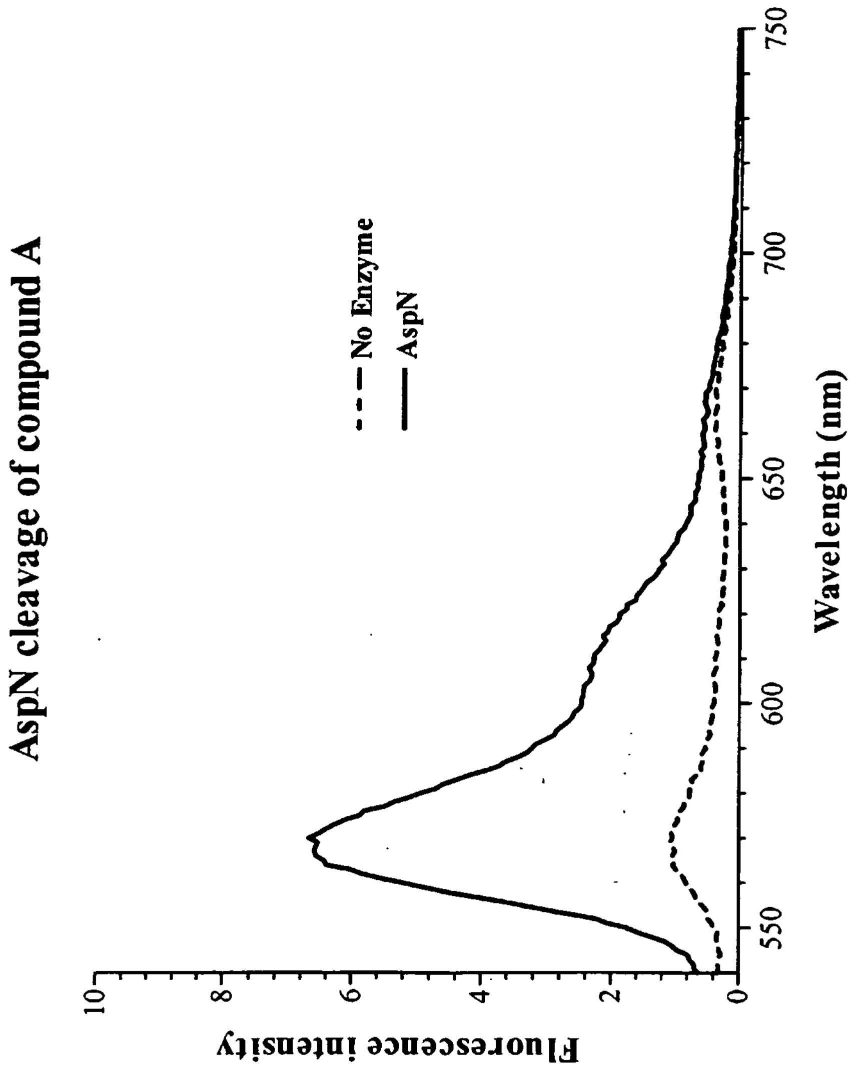
AspN cleavage of compound A
No Enzyme
AspN
Fluorescence intensity
0
2
4
6
8
10
Wavelength (nm)
550
600
650
700
750

Figure 3